# EUROPEAN PATENT SPECIFICATION

(11) **EP 0 378 910 B1**
(45) Date of publication and mention of the grant of the patent: **31.08.1994**
(21) Application number: 89312769.6
(22) Date of filing: 07.12.1989
(51) Int. Cl.: C07D 233/58, C08G 59/50

(54) **Method for the preparation of imidazoles**
Verfahren zur Herstellung von Imidazolen
Procédé de préparation d'imidazoles

(30) Priority: 15.12.1988 US 284883; 15.12.1988 US 284884
(43) Date of publication of application: 25.07.1990
(73) Proprietor: TEXACO DEVELOPMENT CORPORATION, White Plains, New York 10650 (US)
(72) Inventor: Speranza, George Phillip, Austin, Texas 78757 (US); Wei-Yang, Su, Austin, Texas 78759 (US)
(74) Representative: Brock, Peter William

(56) References cited:
- EP-A- 0 064 820
- EP-A- 0 194 984
- CH-A- 193 338
- DE-A- 3 009 605
- DE-A- 3 009 631
- US-A- 2 399 601
- US-A- 3 152 998
- US-A- 3 629 278
- US-A- 3 763 098
- US-A- 4 410 706
- US-A- 4 436 892

## Description

This invention concerns improved methods for the preparation of imidazoles. More specifically, this invention concerns a method for the preparation of imidazoles by the catalytic dehydrogenation of imidazolines. In one embodiment, the imidazolines can be synthesized from non-heterocyclic materials and converted directly into the imidazoles without isolation of the intermediate imidazoline.

Imidazoles and imidazole derivatives may be used as hardening agents for epoxy resins. They provide long pot life, high heat distortion temperatures, economical performance based on low PHR requirements and lower toxicity than amines.

Imidazoles are useful accelerators for anhydride cure and bisphenol-A cure of epoxy resins. The imidazole ring can be utilized in numerous reactions and the derivatives can be used for specialty epoxies. For example, delayed action can be obtained by acylating imidazoles with polychlorinated benzoyl chloride. See US-A-4 436 892. 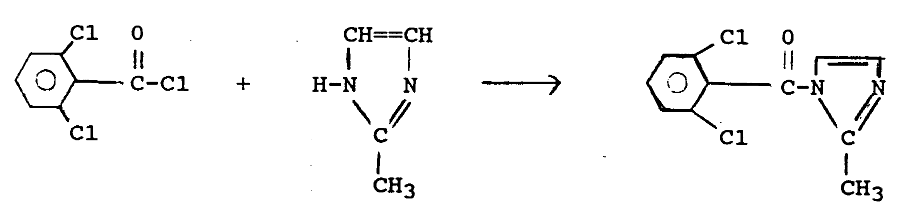
Imidazoles are characterized by their essentially aromatic character, high boiling points, and good stability to oxidizing and reducing agents. They undergo typical aromatic reactions such as nitration, chlorination and diazo coupling. The 2-alkyl and 2-alkenyl imidazoles show a strong detergent and emulsifier action in petroleum oils and they protect metals in contact with such oils.

The long chain 2-alkyl imidazoles are surface active agents and the corresponding quaternary compounds show typical germicidal properties of cationic surfactants. Some other uses include fabric softeners (Europa Chemie April 30, 1983, p. 195), antifungal agents (World Pharmaceutical News, April 20, 1981, p. 15), photosensitive product reagents for the preparation of urethane resins, catalysts for the preparation of polyesters and polyurethanes, plastic additives, anticonvulsant drugs, and antimicrobial agents. Certain imidazoles are effective in the control of anthrenus flavipies, an insect pest of wool.

Early synthesis of imidazoles involved the reaction of 1,2-dicarbonyl compounds with ammonia and aldehydes to produce low yields according to the equation: 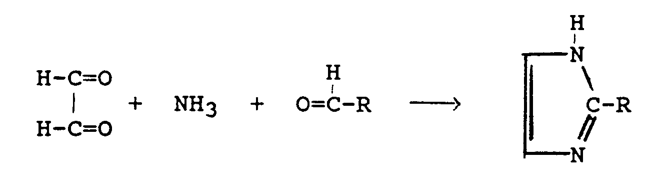
The yields could be increased if the reaction was carried out in organic acid with ammonium acetate. Radziszewski, R. Ber, 15, 2706 (1882).

Imidazole oxalate, fumarate, adipate, phthalate and 4-methylimidazole, 4,5-dimethylimidazole and 2-isopropylimidazole have been made from the α,β-dicarbonyl compounds, as demonstrated in U. S. Patent No. 3,715,365.

Werdenhazen, R., and Rienacker, H., Ber. 72, 57 (1939) demonstrated the production of imidazoles from α-hydroxyketones under the influence of ammoniacal cupric acetate and aldehydes represented by the following: 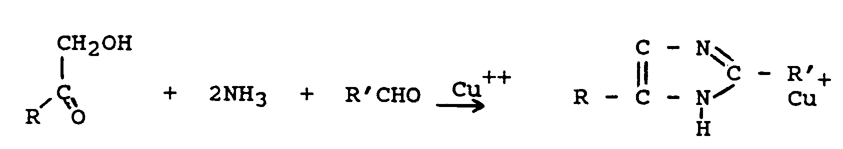
In "New Methods of Preparative Organic Chemistry", Vol. 3, p. 241, Academic Press. N.Y., 1964, H. Budereck, et al. describe another method involving formamide synthesis according to the equation: 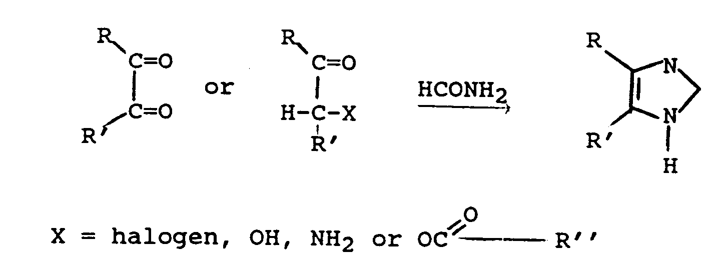
Some 2-methylimidazoles have been prepared using acetamides; however, yields were reduced when amides other than formamide were used.

One of the more attractive methods for producing imidazoles is discussed in U. S. Patent No. 2,891,966. This involved the reaction of a 1,2-diamine with carboxylic acids. For instance, ethylenediamine was admixed with a slight excess of acetic acid, permitting an exothermic rise which dissolved the reagents. The homogeneous solution was cooled to about 100°C, then charged through a continuous reactor equipped with a preheater section (a reactor section filled with platinum). Then the reactor was heated to 430°C and hydrogen added. The vaporous reaction effluent was condensed to obtain crystalline 2-methyl imidazole.

A French patent describes the purification of 2-methyl imidazole by codistilling it with 1- or 2-methylnaphthalene and then washing with pentane or toluene. See French P. 1,362,689 (1964).

In other work, the diamine is converted to 2-alkyl imidazoline, and then dehydrogenated to the corresponding imidazole compound by dehydrogenation over a nickel catalyst. For example see U. S. Patent No. 2,399,601 and U. S. Patent No. 2,404,299 on the preparation of imidazoles by heating imidazolines with Raney nickel catalysts. The reactions were carried out at 225-235°C. The yields were not reported in some cases or varied widely. In these patents the intermediate imidazoline had to be isolated.

H. A. Green, of Air Products has demonstrated that 1,2-diamines can be reacted with aldehydes and then heated over a platinum-alumina catalyst at 370°C to give imidazoles. In the case of ethylenediamine and propionaldehyde a 56% yield of 2-ethyl imidazole was obtained. See U. S. Patent No. 3,037,028, May 29, 1962.

In U. S. Patent No. 3,037,028, using another vapor phase reaction, Green demonstrated that imidazole could be obtained from ethylenediamine and formamide using a large volume of hydrogen. See also U. S. Patent No. 3,255,200. The catalyst used was platinum-on-alumina and alumina or cobalt molybdate were shown to be ineffective. Treating ethylenediamine with methyl formate at 25-30°C gave 98.5% diformyl derivate.

In Ger. Offen. DE 3,009,605, diformyl derivate was passed with nitrogen over 6:14 NiO:MoO₃ at 400°C to give 65.7% imidazole with 99.3% conversion. The yield remained constant after 250 hours use of the catalyst.

Imidazolines can be dehydrogenated to imidazoles at 250-500°C over MoO₃ and NiO and/or CoO and Al₂O₃, SiO₂ and/or alkaline silicate catalysts. See DE 3,009,631. This reaction has been used to make a variety of 2-alkylimidazoles substituted with long chain fatty acids. As noted, the minimum temperature requirement is 250°C.

Another route to imidazoles involved the reaction of a nitrile with a diamine over a copper salt to give imidazolines which were then dehydrogenated over an aluminum-zinc oxide catalyst to give imidazoles, involving two distinct steps. See DE 3,236,598-A to BASF.

In U.S. Patent No. 4,409,389, hexamethylene-tetramine was reacted with formamide at 140°C to give a bis-formamide. The bis-formamide with dicarbonyl compounds and 2 moles of mineral acid yielded imidazole acid salts. 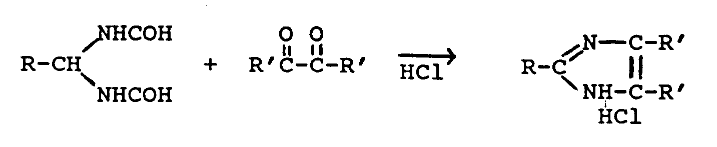
In Ger. Offen. 1,952,991, imidazole was made in 53% yield by passing a solution of ethylenediamine and formic acid over a Cd-Cu chromite catalyst at 480°C. This method required quite high temperatures and the yield was very moderate.

Imidazolines have been dehydrogenated with sulfur and manganese dioxide.

It is noted that most methods for producing imidazoles which are found in the art require higher temperatures and the yields reported are not as high as would be desirable. Some require catalysts which are expensive and would be a deterrent to commercial use. Most procedures require several steps, including isolation of an intermediate imidazoline and subsequent dehydrogenation to the imidazole.

It would be a substantial advance in the art if high yields of imidazoles could be prepared from readily available reactants such as carboxylic acids and diamines without the need to isolate the imidazoline intermediate. Such a process would be extremely attractive commercially.

EP-A-0194984 discloses a class of 4(5)-[2,2-diphenylethyl]- or 4(5)-[2,2-diphenylethenyl]-substituted imidazoles which have α₂- blocking and/or anti-convulsive properties. They can be synthesized by a variety of reaction schemes, including ones which involve dehydrogenation of an imidazoline ring to imidazole using a nickel-containing catalyst.

CH-A-193338 discloses the dehydrogenation of 2-methylimidazoline to 2-methylimidazole using freshly-reduced, finely-divided nickel.

US-A-3629278 discloses copper catalysts for the dehydrogenation of imidazolines. A number of catalysts are mentioned including copper chromite and cadmium copper chromite.

US-A-4410706 also discloses copper chromite for such a reaction, or alteratively one or more of molybdenum, iron, cobalt, zinc or chromium on an oxide support, such as alumina or silica.

It is noted that most methods for producing imidazoles which are found in the art require higher temperatures and the yields reported are nowhere near 99%. Further one does not observe the higher yields and mild temperatures in the same work. For instance, no previously used process for producing imidazoles allows for as great as 99% yield or higher with almost no by-products using temperatures of around 200°C or less.

It would be a substantial advance in the art if imidazoles could be prepared in essentially quantitative yields using mild conditions. Such a process would be particularly attractive economically if essentially no by-products were formed.

This invention describes a selective process for the preparation of imidazoles. According to the invention, imidazolines can be reacted over dehydrogenation catalysts comprising nickel in combination with copper and/or chromium. The imidazole products can be derived in high yield at mild conditions without the formation of by-products.

The imidazolines which are reacted over the dehydrogenation catalyst preferably are of the following structure: 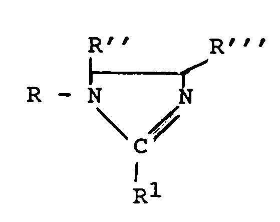
where R is H or an alkyl group containing 1-18 carbon atoms; R¹ is H, an aromatic or an alkyl group containing 1 to 17 carbon atoms; R˝ is H or an alkyl group containing 1-4 carbon atoms; and R‴ is H or an alkyl group containing 1-4 carbon atoms.

Suitable imidazolines can be obtained from several sources. For example, imidazolines can be produced from organic acids and diamines. Examples include 2-heptadecenyl-1-isopropylimidazoline prepared from oleic acid and N-isopropylethylene diamine, and 1-isopropyl-2-methyl imidazoline prepared from acetic acid and N-isopropylethylenediamine.

One embodiment of this invention provides a method for preparation of imidazoles by dehydrogenation of imidazolines in the presence of a catalyst, the imidazoline at a temperature of 160 to 300°C and a pressure of atmospheric to 500 psig (3.55 Mpa).

The catalyst comprises nickel-copper, nickel-chromium or nickel-copper-chromium.The imidazoline may for example have the structure: 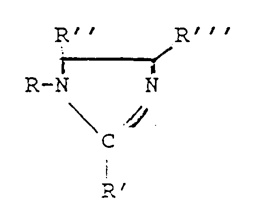
wherein R is H or an alkyl group containing 1 to 18 carbon atoms, R′ is H or an aromatic or alkyl group containing 1 to 17 carbons, R˝ is H or an alkyl group containing 1 to 4 carbon atoms, and R‴ is H or an alkyl group containing 1 to 4 carbon atoms.

In one embodiment the imidazoline is generated in situ by reaction between a diamine and an organic carboxylic acid or nitrile, and is converted directly without isolation into the imidazole. The diamine preferably has the formula 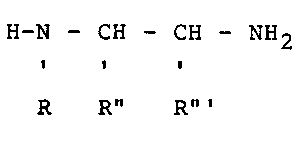
and the carboxylic acid or nitrile has the formula:

R'CO₂H or R''CN

Other imidazolines which provide suitable reactants in the process of this invention include 1-isopropyl-2-methylimidazoline, 2,4-dimethylimidazoline, imidazoline, 2-methylimidazoline and 1-methylimidazoline.

The catalyst employed for dehydrogenation of imidazolines comprises nickel in combination with copper and/or chromium. In other work nickel catalysts have been used as dehydrogenation catalysts, however, it has been surprisingly discovered in the instant invention that the use of one or more additional transition metals in combination with nickel allows the use of much lower temperatures and permits much higher conversions and yields with very minimal or no by-products. These features are very attractive for commercial reasons.

The quantity of nickel compound and copper or chromium employed in the catalyst may vary. The reaction proceeds when employing as little as 50 per cent of nickel, basis the total weight of the catalyst. The percentage of copper and/or chromium should preferably be 2 to 30%. From 70 to 80 wt% of nickel in conjunction with from 20 to 30 of copper and/or chromium is generally desirable. A preferred catalyst is described in US-A-3152998, comprising to to 85 mol percent of nickel, 14 to 37 mol percent of copper and 1 to 5 mol percent of chromium.

Possible catalysts can comprise:
70 to 98 weight % of nickel in combination with 2 to 30 weight % of copper, chromium, or a combination thereof;
80 to 99 weight percent of nickel and 1 to 20 weight percent chromium;
95 to 99.8 weight percent of nickel and 0.2 to 5 weight percent of chromium; and
60 to 80 weight % of nickel, 14 to 37 weight % of copper, and 1 to 5 weight % of chromium.

In the alternative embodiment, involving generation of the imidazoline in situ, the reaction scheme follows the course: 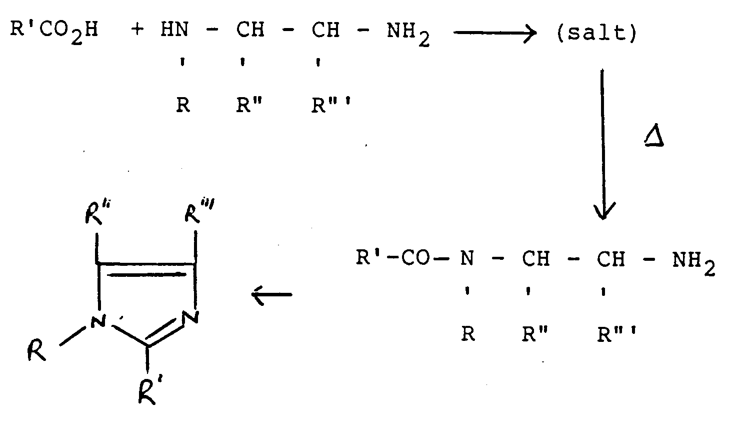
R, R′, R" and R‴ having the meanings given above.

As stated, the reactants useful in the invention are diamines and organic acids. The diamines which will work include alkylene diamines. The preferred diamines are ethylenediamines of the formula 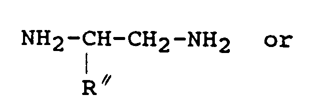
N-alkylethylenediamines of the formula 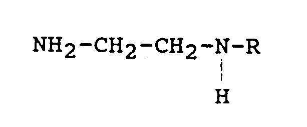
Examples include N-methyl, N-isopropyl, N-isobutyl, N-methylisobutyl ethylenediamines and 1,2-propylenediamine derivatives.

Suitable organic acids are primarily aliphatic carboxylic acids having 1 to 18 carbon atoms. Examples include propionic, isobutyric, acetic acid, lauric acid and myristic acid. Aromatic carboxylic acids may also be used.

The organic acid and diamine are simply heated to form an amide which is subsequently passed over the catalyst at about 200°C to give the imidazoles without the necessity of isolating the intermediate imidazoline.

In other work metal catalysts have been used as dehydrogenation catalysts, in two-step syntheses of imidazoles; however, it has been surprisingly discovered in the instant invention that the imidazoles can be prepared from the aminoethylamide reaction product without the isolation of the imidazoline intermediate. In addition almost quantitative yields are obtained when N-alkyl ethylenediamines are used. Those skilled in the art will recognize how desirable such features are for commercial reasons.

The temperature range which can usefully be employed is variable depending upon other experimental factors, including the pressure and the choice of particular species of catalyst among other things. The range of operability is from 150°C to 300°C. A narrow range of 180° to 250°C is preferred and reflects significantly milder temperatures than previously used in the art for similar reactions.

Pressures of 0 to 500 psig (3.55 MPa) can be used. Substantial yields are realized using atmospheric pressure.

As noted specific products demonstrated in this invention include:
1-3′-pentyl-2-methylimidazole;
1-4′-methyl-2′-pentyl-2-methylimidazole
1-isopropyl-2-methylimidazole
1-isopropyl-2-undecylimidazole
1-isopropyl-2-tridecylimidazole
These imidazole and simple imidazole derivatives are being used more as hardening agents for epoxy resins. They provide long pot life, high heat distortion temperatures, economical performance based on low PHR requirements and lower toxicity than amines. They are useful accelerators (catalysts) for anhydride cure and bisphenol -A cure of epoxy resins. The imidazole ring can be utilized in numerous reactions and these derivatives can be used for specialty epoxies.

The process can be conducted in a kettle, tubular reactor or a glass reactor. The catalyst may be initially introduced into the reaction zone batchwise, or it may be continuously or intermittently introduced into such a zone during the course of the synthesis reaction. Operating conditions can be adjusted to optimize the formation of the desired imidazole products. The products are recovered preferably by fractional distillation.

The products have been identified by one or more of the following analytical procedures, viz, gas-liquid chromatograph (glc), infrared (IR), nuclear magnetic resonance (nmr) and mass spectra or a combination of these techniques. Analyses have, for the most part, been by parts in weight; all temperatures are in degrees centigrade and all pressures in pounds per square inch guage (psig). The selectivity means the moles of imidizole produced divided by moles of reactants.

Various embodiments of the process of this invention are illustrated in the following Examples.
Harshaw^{(R)} Ni-2715 catalyst is a proprietary Ni-Cu-Cr catalyst.

### EXAMPLE 1

### Preparation of 1-Isopropyl-2-methylimidazole

A 100-ml three-necked flask equipped with a thermometer, condenser, magnetic stirrer, and nitrogen inlet was charged with 1-isopropyl-2-methylimidazoline (50g, 0.4 mole) and nickel-copper-chromium catalyst (5.0g) (see Ex 1 of U. S. Patent No. 3,152,998). The reaction was carried out at 200°C with stirring and under a nitrogen atmosphere for about 5 hours. GLC analysis showed that more than 99% of the starting imidazoline had been converted to give the corresponding imidazole with 98.5% selectivity. The product was fractionally distilled under reduced pressure (80°C at 1.2 mm Hg) to give a clear, colorless liquid in about 98% yield.

### EXAMPLE 2

### Preparation of 2,4(5)-Dimethylimidazole

The procedure of Example 1 was followed except that 58.9g of 2,4-dimethylimidazoline and 5.8g of the same catalyst used in Example 1 were charged. GLC analysis showed that a 96% yield of 2,4(5)-dimethylimidazole and 3% yield of imidazole isomers were obtained.

### EXAMPLE 3

### Preparation of 2-Heptadecenyl-1-isopropylimidazole

A 100-ml three-necked flask equipped with a thermometer, condenser, magnetic stirrer and nitrogen inlet was charged with 50g of 2-heptadecenyl-1-isopropylimidazoline (prepared from oleic acid and N-isopropylethylene diamine) and 5.0g of nickel-copper-chromium catalyst as used in Example 1. The reaction was carried out at 200°C with stirring and under a nitrogen atmosphere for about 7 hours. The product was analyzed by C¹³, NMR and IR and found to be almost all 2-heptadecenyl-1-isopropyl imidazole.

### EXAMPLE 4

### Preparation of 2-Heptadecenylimidazole

The procedure of Example 3 was followed except that 48.9g of 2-heptadecenyl imidazoline (prepared from oleic acid and ethylenediamine) and 5.0g of the same nickel-copper-chromium catalyst were charged. The product was analyzed by C¹³, NMR and IR and found to be almost all 2-heptadecenylimidazole. The yield was almost quantitative.

### COMPARATIVE EXAMPLE 1

### Cobalt Catalyst

The procedure of Example 1 was followed except that 26.2g of 1-isopropyl-2-methylimidazoline and 3.0g of a cobalt hydrogenation catalyst was charged. GLC analysis show that a 14% yield of 1-isopropyl-2-methylimidazole was obtained with 19% conversion of imidazoline.

### EXAMPLE 5

### Raney Ni-Cr

The procedure of Example 1 was followed except that 50.0g of 1-isopropyl-2-methylimidazoline and about 5g of Raney Nickel-chromium catalyst was charged. GLC analysis showed that 98% yield of 1-isopropyl-2-methylimidazole was obtained with >99% conversion of imidazoline.

### EXAMPLE 6

### Preparation of 1-3′-Pentyl-2-methylimidazole

A 250-ml three-necked flask equipped with a thermometer, condenser, stirrer, and nitrogen inlet was charged with N-3′-pentylethylenediamine (104g, 0.8 mol). Acetic acid (48g, 0.8 mol) was added dropwise over a 10 minute period. After finishing the acid addition, the mixture was heated to 130-140°C for two hours. The reaction mixture was cooled to room temperature. A Dean-Stark trap was added and Harshaw Ni-2715 catalyst was also added. The mixture was heated to 200°C for five hours. The resulting reaction mixture was filtered and about 118g of crude product was obtained. GLC analysis showed that the crude product contained 99% of 1-3′-pentyl-2-methyl-imidazole. The product was fractionally distilled under reduced pressure (92°C at 0.5 mm Hg) to give a clear, colorless liquid in about 91% yield.

### EXAMPLE 7

### Preparation of 1-4′-methyl-2′-pentyl-2-methyl-imidazole

The procedure of Example 6 was followed except that 100.8g (0.7 mol) of N-4′-methyl-2′-pentylethylenediamine and 42g (0.7 mol) of acetic acid were used. GLC analysis showed that the crude product contained 99% of 1-4′-methyl-2′-pentyl-2-methylimidazole. The product was fractionally distilled under reduced pressure (85°C at 0.35 mm Hg) to give a clear, colorless liquid in about 89% yield.

### EXAMPLE 8

### Preparation of 1-Isopropyl-2-methylimidazole

The procedure of Example 6 was followed except that 33.1g (0.32 mol) of N-isopropylethylenediamine, 19.4g (0.32 mole) of acetic acid and 5.9g of Harshaw Ni-2715 catalyst were used. About 37.6g of crude product was recovered. GLC analysis showed that the crude product contained 98% of 1-isopropyl-2-methylimidazole.

### EXAMPLE 9

### Preparation of 1-Isopropyl-2-undecylimidazole

A 100-ml three-necked flask equipped with a thermometer, Dean-Stark trap, stirrer, and nitrogen inlet was charged with 26.0g of N-2′-isopropylaminoethyldodecanamide (prepared from lauric acid and N-isopropylethylenediamine) and 3.7g of Harshaw Ni-2715 catalyst. The mixture was heated to 200°C for five hours. The resulting reaction mixture was filtered and about 20.3g of light-green liquid product was obtained. The IR spectrum of this product indicates it to be that of 1-isopropyl-2-undecylimidazole.

### EXAMPLE 10

### Preparation of 1-Isopropyl-2-tridecylimidazole

The procedure of Example 4 was followed except that 32.2g of N-2′-isopropylaminoethyltetradecanamide (prepared from myristic acid and N-isopropylethylenediamine) and 4.4g of Harshaw Ni-2715 catalyst were used. About 25.5g of light green liquid product was obtained. The IR spectrum of this product indicated it to be 1-isopropyl-2-tridecylimidazole.

## Claims

1. A method for preparation of imidazoles by dehydrogenation of imidazolines in the presence of a catalyst, characterised in that the imidazoline is contacted with a catalyst comprising nickel-copper, nickel-chromium or nickel-copper-chromium, at a temperature of 160 to 300°C and a pressure of atmospheric to 500 psig (3.55 MPa)

2. A method according to Claim 1 characterised in that the imidazoline has the structure: 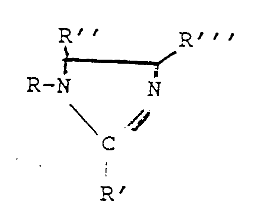 wherein R is H or an alkyl group containing 1 to 18 carbon atoms, R′ is H or an aromatic or alkyl group containing 1 to 17 carbons, R˝ is H or an alkyl group containing 1 to 4 carbon atoms, and R‴ is H or an alkyl group containing 1 to 4 carbon atoms.

3. A method according to Claim 1 characterised in that the imidazoline is generated in situ by reaction between a diamine and an organic carboxylic acid or nitrile, and is converted directly without isolation into the imidazole.

4. A method according to Claim 3 characterised in that the diamine has the formula: 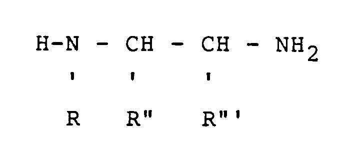 and the carboxylic acid or nitrile has the formula:
R′CO₂H or R′CN
wherein
R, R′, R˝ and R‴ have the meanings given in Claim 1

5. A method according to any of the Claims 1 to 4 characterised in that the catalyst comprises from 70 to 98 weight percent of nickel in combination with from 2 to 30 weight percent of copper, chromium or a combination thereof.

6. A method according to any one of Claims 1 to 4 characterised in that the catalyst comprises 80 to 99 weight percent of nickel and 1 to 20 weight percent of chromium.

7. A method according to any one of Claims 1 to 4 characterised in that the catalyst comprises 60 to 80 weight percent of nickel, 14 to 37 weight percent of copper and 1 to 5 weight percent of chromium.

8. A method according to any one of Claims 1 to 4 characterised in that the catalyst comprises 95 to 99.8 weight percent of nickel and 0.2 to 5 weight percent of chromium.

## Patentansprüche

1. Ein Verfahren zur Herstellung von Imidazolen durch Dehydrierung von Imidazolinen in der Gegenwart eines Katalysators, dadurch gekennzeichnet, daß das Imidazolin mit einem Katalysator, der Nickel-Kupfer, Nickel-Chrom oder Nickel-Kupfer-Chrom umfaßt, bei einer Temperatur von 160 bis 300°C und einem Druck von atmosphärisch bis 500 psig (3,55 MPa) in Kontakt gebracht wird.

2. Ein Verfahren nach Anspruch 1, dadurch gekennzeichnet, daß das Imidazolin die Struktur besitzt: 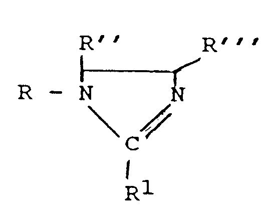 worin R H oder eine Alkylgruppe, die 1 bis 18 Kohlenstoffatome enthält, ist, R' H oder eine aromatische oder Alkylgruppe, die 1 bis 17 Kohlenstoffe enthält, ist, R'' H oder eine Alkylgruppe, die 1 bis 4 Kohlenstoffatome enthält, ist und R''' H oder eine Alkylgruppe, die 1 bis 4 Kohlenstoffatome enthält, ist.

3. Ein Verfahren nach Anspruch 1, dadurch gekennzeichnet, daß das Imidazolin in situ durch Reaktion zwischen Diamin und einer organischen Carbonsäure oder einem Nitril erzeugt wird und direkt ohne Isolierung in das Imidazol überführt wird.

4. Ein Verfahren nach Anspruch 3, dadurch gekennzeichnet, daß das Diamin die Formel besitzt: 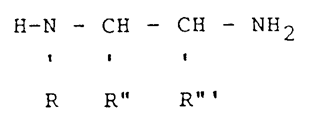 und die Carbonsäure oder das Nitril die Formel besitzt:
R'CO₂H oder R'CN
worin
R, R', R'' und R''' die in Anspruch 1 angegebenen Bedeutungen haben.

5. Ein Verfahren nach einem der Ansprüche 1 bis 4, dadurch gekennzeichnet, daß der Katalysator von 70 bis 98 Gew.-% Nickel in Kombination mit von 2 bis 30 Gew.-% Kupfer, Chrom oder einer Kombination derselben umfaßt.

6. Ein Verfahren nach einem der Ansprüche 1 bis 4, dadurch gekennzeichnet, daß der Katalysator 80 bis 99 Gew.-% Nickel und 1 bis 20 Gew.-% Chrom umfaßt.

7. Ein Verfahren nach einem der Ansprüche 1 bis 4, dadurch gekennzeichnet, daß der Katalysator 60 bis 80 Gew.-% Nickel, 14 bis 37 Gew.-% Kupfer und 1 bis 5 Gew.-% Chrom umfaßt.

8. Ein Verfahren nach einem der Ansprüche 1 bis 4, dadurch gekennzeichnet, daß der Katalysator 95 bis 99,8 Gew.-% Nickel und 0,2 bis 5 Gew.-% Chrom umfaßt.

## Revendications

1. Procédé de préparation d'imidazoles par déshydrogénation d'imidazolines en présence d'un catalyseur, caractérisé en ce que l'imidazoline est mise en contact avec un catalyseur comprenant du nickel-cuivre, du nickel-chrome ou du nickel-cuivre-chrome, à une température de 160 à 300 °C et à une pression allant de la pression atmosphérique à 500 psig (3,55 MPa).

2. Procédé selon la revendication 1, caractérisé en ce que l'imidazoline a la structure : 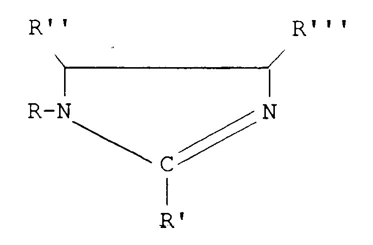 où R est H ou un groupe alkyle contenant 1 à 18 atome(s) de carbone, R' est H ou un groupe aromatique ou alkyle contenant 1 à 17 carbone(s), R'' est H ou un groupe alkyle contenant 1 à 4 atome(s) de carbone, et R''' est H ou un groupe alkyle contenant 1 à 4 atome(s) de carbone.

3. Procédé selon la revendication 1, caractérisé en ce que l'imidazoline est produite *in situ* par une réaction entre une diamine et un acide carboxylique organique ou un nitrile, et qu'elle est directement convertie sans isolement en imidazole.

4. Procédé selon la revendication 3, caractérisé en ce que la diamine a la formule : 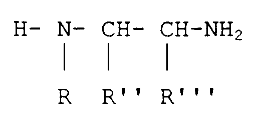 et en ce que l'acide carboxylique ou le nitrile a la formule :
R'CO₂H ou R'CN
où
R, R', R'' et R''' ont les significations données dans la revendication 1.

5. Procédé selon l'une quelconque des revendications 1 à 4, caractérisé en ce que le catalyseur comprend de 70 à 98 % en poids de nickel en combinaison avec 2 à 30 % en poids de cuivre, de chrome, ou d'une combinaison de ceux-ci.

6. Procédé selon l'une quelconque des revendications 1 à 4, caractérisé en ce que le catalyseur comprend 80 à 99 % en poids de nickel et 1 à 20 % en poids de chrome.

7. Procédé selon l'une quelconque des revendications 1 à 4, caractérisé en ce que le catalyseur comprend 60 à 80 % en poids de nickel, 14 à 37 % en poids de cuivre et 1 à 5 % en poids de chrome.

8. Procédé selon l'une quelconque des revendications 1 à 4, caractérisé en ce que le catalyseur comprend 95 à 99,8 % en poids de nickel et 0,2 à 5 % en poids de chrome.
